(19) Europäisches Patentamt
European Patent Office
Office européen des brevets

(11) EP 4 269 467 A1

(12) **EUROPEAN PATENT APPLICATION**
published in accordance with Art. 153(4) EPC

(43) Date of publication:
**01.11.2023 Bulletin 2023/44**

(21) Application number: **21910769.5**

(22) Date of filing: **21.12.2021**

(51) International Patent Classification (IPC):
***C08G 65/333*** *(2006.01)* ***A61K 31/197*** *(2006.01)*
***A61K 31/77*** *(2006.01)* ***A61P 1/04*** *(2006.01)*
***A61P 41/00*** *(2006.01)* ***C08L 71/02*** *(2006.01)*

(52) Cooperative Patent Classification (CPC):
**A61K 31/197; A61K 31/77; A61P 1/04; A61P 41/00; C08G 65/333; C08L 71/02**

(86) International application number:
**PCT/JP2021/047282**

(87) International publication number:
**WO 2022/138626 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 JP 2020216704**

(71) Applicants:
- **Sanyo Chemical Industries, Ltd.**
  **Kyoto-shi, Kyoto 605-0995 (JP)**
- **Juntendo Educational Foundation**
  **Tokyo 113-8421 (JP)**

(72) Inventors:
- **MURAKAMI, Yuta**
  **Kyoto-shi, Kyoto 605-0995 (JP)**
- **NAKAI, Kenichiro**
  **Kyoto-shi, Kyoto 605-0995 (JP)**
- **OTA, Nozomi**
  **Kyoto-shi, Kyoto 605-0995 (JP)**
- **MOTOFUJI, Shihei**
  **Kyoto-shi, Kyoto 605-0995 (JP)**
- **NAKAMURA, Tetsuya**
  **Tokyo 113-8421 (JP)**
- **MATSUMOTO, Yuka**
  **Tokyo 113-8421 (JP)**

(74) Representative: **Mewburn Ellis LLP**
**Aurora Building**
**Counterslip**
**Bristol BS1 6BX (GB)**

(54) **COMPOUND, COMPOSITION, BIOTISSUE EMBRITTLEMENT AGENT, BIOTISSUE SURFACE DETACHMENT METHOD, AND BIOTISSUE DETACHMENT KIT**

(57) Provided is a compound (Z) represented by the formula (1) or (2) below. In the formulas (1) and (2), Xs in each of the formulas (1) and (2) are each independently a carboxy group, a carboxylate group, or a monovalent group represented by the formula (3); at least one of Xs in each of the formulas (1) and (2) is a carboxy group or a carboxylate group; and at least one of Xs in each of the formulas (1) and (2) is a monovalent group represented by the formula (3) $-C(O)-Y-(AO)_n-R$ (3). In the formula (3), -Y- is -O-, -NH-, or -S-; A is a C2-C4 alkylene group; R is a hydrogen atom or a C1-C15 monovalent hydrocarbon group in which a hydrogen atom is optionally substituted with a C1-C10 alkoxy group; n is an integer of 4 to 1000; at least one of n As is an ethylene group; and when the formulas (1) and (2) each have multiple monovalent groups represented by the formula (3), each of -Y-, A, R, and n is the same or different among the monovalent groups.

EP 4 269 467 A1

[Chem. 1]

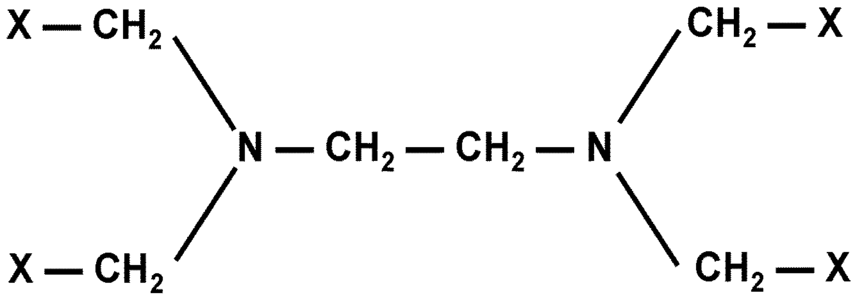

(1)

[Chem. 2]

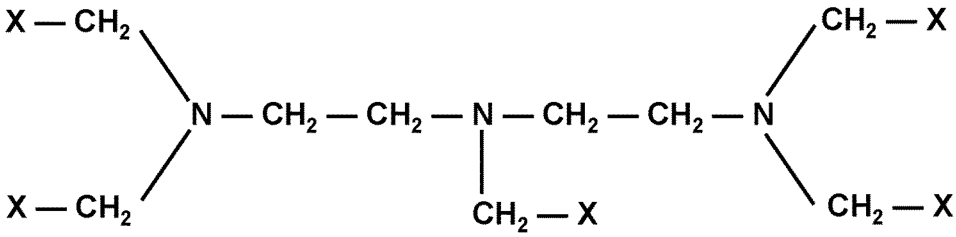

(2)

## Description

TECHNICAL FIELD

The present invention relates to a compound, a composition, a biological tissue embrittling agent, a method for detaching a biological tissue surface, and a biological tissue detachment kit.

BACKGROUND ART

With the development of regenerative medicine technology, processing of biological tissue surfaces and transplantation of cells are about to be put into practical use. Such techniques are useful for removal of abnormal tissue and repair of refractory inflammatory tissue. The short bowel syndrome, one of refractory intestinal diseases, caused by extensive resection of the small intestine may induce residual intestinal dysfunction that may require long-term total parenteral nutrition (TPN) or small bowel transplantation. Although small bowel transplantation is sometimes performed, postoperative rejection and donor shortage are critical issues, and no practical treatment method has been established.

Breakthrough treatments have been required for the short bowel syndrome, and many studies have been conducted in the field of regenerative medicine. In particular, use of intestinal organoids for regeneration of the function of small intestine has attracted attention. For example, Non-Patent Literatures 1 to 3 have reported that mouse colonic epithelium is detached through the tissue embrittlement effect obtained by using ethylenediaminetetraacetic acid (EDTA) as a chelating agent and through subsequent mechanical stimulation, and small intestinal organoids are then transplanted thereto and maintained in the animal for a long period of time.

Non-Patent Literature 3 was issued after the priority date of the international patent application of the present application.

CITATION LIST

- Non-Patent Literature

Non-Patent Literature 1: Genes and Development, 2014, Vol. 28, pp. 1752-1757
Non-Patent Literature 2: Cell Stem Cell, 2018, Vol. 22, pp. 1-6
Non-Patent Literature 3: Nature, 2021, Vol. 592, 1 April, pp. 99-104

SUMMARY OF INVENTION

- Technical Problem

The methods of the non-patent literatures however may cause entry of EDTA, which has leaked into the blood during colonic epithelium detachment, into the systemic circulation. Thereby, serious side effects such as hypocalcemia may occur. When the mucosal epithelium is detached by applying a chelating agent such as EDTA to the normal mucosal epithelium, such risks are required to be minimized by preventing mucosal damage and preventing transport of the chelating agent into the blood.

The present invention has been made in view of processing and repairing biological tissue in regenerative medicine, and aims to provide a material effective as a biological tissue embrittling agent and safe for biological body, and also provide a method of safely detaching a biological tissue surface.

- Solution to Problem

As a result of extensive studies to solve the above problems, the present inventors arrived at the present invention.

That is, the present invention relates to a compound (Z) represented by the formula (1) or (2) below; a composition containing the compound (Z); a biological tissue embrittling agent containing the compound (Z); a method for detaching a biological tissue surface, including: bringing the compound (Z) into contact with biological tissue to embrittle a surface of the biological tissue; and detaching cell tissue from the embrittled surface of the biological tissue; and a biological tissue detachment kit including the biological tissue embrittling agent.

The formulas (1) and (2) are as follows.

[Chem. 1]

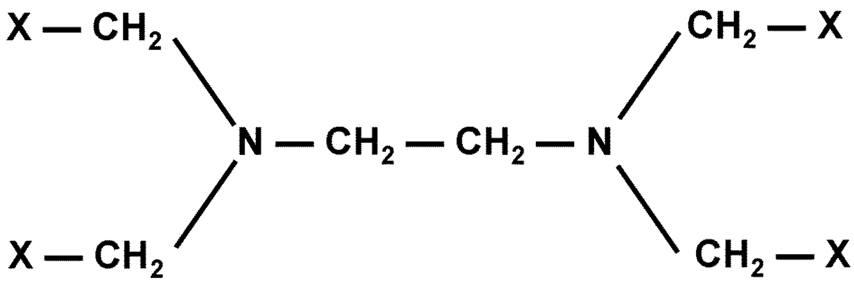

(1)

[Chem. 2]

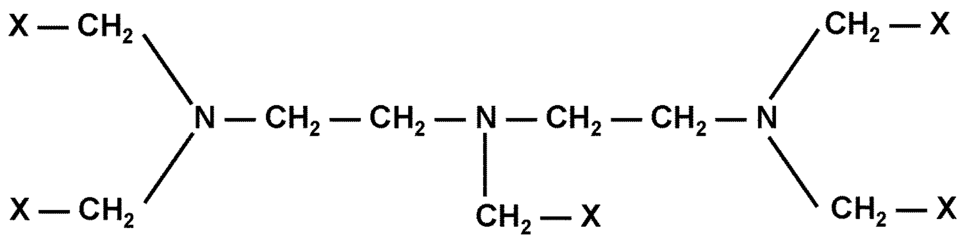

(2)

Xs in each of the formulas (1) and (2) are each independently a carboxy group, a carboxylate group, or a monovalent group represented by the formula (3); at least one of Xs in each of the formulas (1) and (2) is a carboxy group or a carboxylate group; and at least one of Xs in each of the formulas (1) and (2) is a monovalent group represented by the formula (3):

$$-C(O)-Y-(AO)_n-R \qquad (3)$$

wherein -Y- is -O-, -NH-, or -S-; A is a C2-C4 alkylene group; R is a hydrogen atom or a C1-C15 monovalent hydrocarbon group in which a hydrogen atom is optionally substituted with a C1-C10 alkoxy group; n is an integer of 4 to 1000; at least one of n As is an ethylene group; and when the formulas (1) and (2) each have multiple monovalent groups represented by the formula (3), each of -Y-, A, R, and n is the same or different among the monovalent groups.

- Advantageous Effects of Invention

The present invention can provide a material effective as a biological tissue embrittling agent and safe for biological body, and can also provide a method of safely detaching a biological tissue surface.

The present invention can also provide a biological tissue embrittling agent useful for processing and repairing tissue in regenerative medicine. Thereby, tissue to be detached and removed can be processed and modified by an easy and highly-safe method. In particular, it is useful for detaching the mucosal epithelium and newly transplanting organoids or the like to the detached sites.

DESCRIPTION OF EMBODIMENTS

The present invention relates to a compound (Z) represented by the formula (1) or (2).

[Chem. 3]

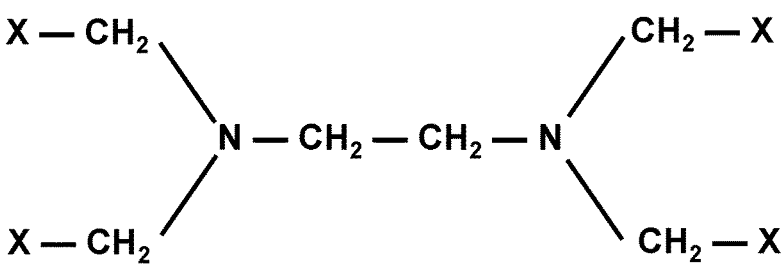

(1)

[Chem. 4]

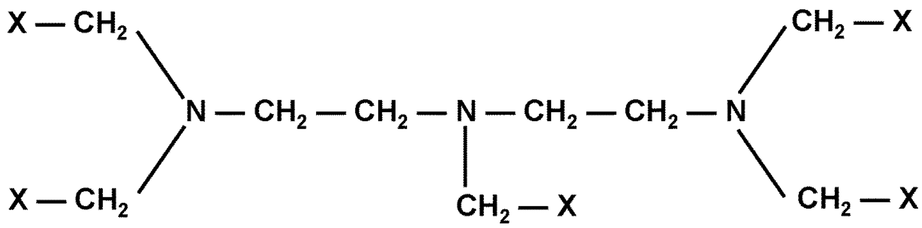

(2)

Xs in each of the formulas (1) and (2) are each independently a carboxy group, a carboxylate group, or a monovalent group represented by the formula (3).

At least one of Xs in each of the formulas (1) and (2) is a carboxy group or a carboxylate group. From the viewpoint of biological tissue embrittlement, at least two of Xs in each of the formulas (1) and (2) are preferably selected from a carboxy group and a carboxylate group, and at least three thereof are more preferably selected from a carboxy group and a carboxylate group.

At least one of Xs in each of the formulas (1) and (2) is a monovalent group represented by the formula (3). From the viewpoint of safety in biological body, at least two of Xs in each of the formulas (1) and (2) are preferably monovalent groups represented by the formula (3) .

Aminopolycarboxylic acid compounds have long been used as chelating agents in industrial applications. "DTPA (skeleton corresponding to the formula (2))" has almost the same chelate-forming ability as "EDTA (skeleton corresponding to the formula (1))". The stability constants (log K) for calcium ion of EDTA and DTPA are respectively 10.9 and 10.7. There is no significant difference in safety between intravenous administration of Ca-EDTA to beagle dogs for one month and intravenous administration of Ca-DTPA to beagle dogs for one month.

Therefore, the compounds represented by formulas (1) and (2) are considered to exhibit the same effects in terms of chelating ability and safety.

When a carboxylate group for X is present in the formulas (1) and (2), the counterion to the carboxylate group may be an alkali metal ion (e.g., sodium ion and potassium ion), an alkaline earth metal ion (e.g., calcium ion), or the like, preferably a sodium ion.

$$-C(O)-Y-(AO)_nR \qquad (3)$$

In the formula (3), -Y- is -O-, -NH-, or -S-, and -Y-is preferably -O-.

The case where -Y- is -NH- or -S- achieves the same chelating effect as the case where Y is -O- because -NH- and -S- are heteroatoms having an unshared lone pair of electrons. Moreover, safety is similar between the case where Y- is -NH- or -S- and the case where -Y- is -O-.

In the formula (3), A is a C2-C4 alkylene group (e.g., an ethylene group, a 1,2- or 1,3-propylene group, a 1,2-, 1,3-, 1,4-, or 2,3-butylene group).

In the formula (3), R is a hydrogen atom or a C1-C15 monovalent hydrocarbon group (e.g., a methyl group, an ethyl group, an iso-propyl group, a tert-butyl group, a neopentyl group, or a tetradecyl group) in which a hydrogen atom is optionally substituted with a C1-C10 alkoxy group.

In the formula (3), n is an integer of 4 to 1000. From the viewpoint of safety in biological body, n is preferably 10 to 500. From the viewpoint of biological tissue embrittlement, n is more preferably 4 to 100, even more preferably 4 to 50.

In the formula (3), at least one of n As is an ethylene group. From the viewpoint of formulation stability and safety in biological body, preferably, not lower than 85% of the number of n As are ethylene groups.

The chelating effects are similar regardless of whether A is any of C2-C4 alkylene groups. The safety is also similar regardless of whether A is any of C2-C4 alkylene groups.

When the formulas (1) and (2) each have multiple monovalent groups represented by the formula (3), each of - Y-, A, R, and n is the same or different among the monovalent groups.

From the viewpoint of biological tissue embrittlement, the total number of moles of carboxy groups and carboxylate groups in the compound (Z) is preferably 0.04 to 14 mmol/g, more preferably 0.04 to 5 mmol/g, based on the weight of the compound (Z).

From the viewpoint of safety in biological body, the compound (Z) preferably has a weight average molecular weight of 500 to 20,000, more preferably 500 to 10,000, even more preferably 2,000 to 10,000.

In a composition containing multiple compounds (Z) each represented by the formula (1) or (2), all the compounds corresponding to the compounds (Z) each preferably have a weight average molecular weight within the above range.

In the present invention, the weight average molecular weight can be measured by the following method.

Device: HLC-8320GPC (Tosoh Corporation)
Column: TSK gel Super AW available from Tosoh Corporation
Detector: RI (Refractive Index)
Eluent: 0.01 M LiBr-DMF
Eluent flow rate: 0.6 mL/min
Column temperature: 40°C
Sample concentration: 0.125 wt%
Injection volume: 20 μL
Reference material: TSK STANDARD POLYETHYLENE OXIDE available from Tosoh Corporation

From the viewpoint of safety in biological body, the compound (Z) preferably has a number average molecular weight of 500 to 20,000, more preferably 500 to 10,000, even more preferably 2,000 to 10,000.
In a composition containing multiple compounds (Z) each represented by the formula (1) or (2), all the compounds corresponding to the compounds (Z) each preferably have a number average molecular weight within the above range.
In the present invention, the number average molecular weight can be measured by the following method.
The number average molecular weight can be measured using $^1$H-NMR. For example, when the compound (Z) is a mixture of a compound represented by the formula (1) containing one monovalent group represented by the formula (3) and a compound represented by the formula (1) containing two monovalent groups represented by the formula (3), where each A is a C2 alkylene group, the number average molecular weight can be calculated by the following method.
Specifically, the number average molecular weight can be calculated by the equations below using the molar ratio ($\alpha$:$\beta$) of the compound represented by the formula (1) containing one monovalent group represented by the formula (3) to the compound represented by the formula (1) containing two monovalent groups represented by the formula (3) and the integral ($\gamma$) of the signal of the methylene group of A in $^1$H-NMR. When the integrals in $^1$H-NMR are calculated, the total integral of signals of two methylene groups between two nitrogen atoms in the formula (1) is set to 4.

$$n_{sum} = \gamma/\{[4 \times \alpha + 8 \times \beta]/(\alpha + \beta)\}$$

$$(\text{Number average molecular weight}) = 44 \times n_{sum} + 292$$

<Conditions of evaluation of $^1$H-NMR>

Solvent: Deuterated DMSO
Apparatus: AVANCE III HD400 (Bruker Japan K.K.)
Frequency: 400 MHz

The compound (Z) can be produced by the following method.
EDTA dianhydride and water (preferably, 1 molar equivalent with respect to EDTA dianhydride) are mixed in an organic solvent (e.g., DMF) at 70°C to 80°C preferably for 2 to 4 hours to obtain EDTA monoanhydride.
Next, the EDTA monoanhydride is combined with a basic compound (e.g., triethylamine and diisopropylethylamine) and a compound represented by the formula (4) and they are mixed at 25°C to 70°C preferably for 2 to 24 hours to obtain the compound (Z). (In this case, the compound (Z) having one monovalent group represented by the formula (3) is obtained as the main product. In the formula (3), -Y- is -O-.)

$HO\text{-}(AO)_nR$ (4)

The letters A, R, and n in the formula (4) are the same as A, R, and n in the formula (3).
The following compounds may be used instead of the compound represented by the formula (4).

$H_2N\text{-}(AO)_n\text{-}R$ (5)

$HS\text{-}(AO)_n\text{-}R$ (6)

When a compound represented by the formula (5) is used, -Y- in the formula (3) is -NH-. When a compound

represented by the formula (6) is used, -Y- in the formula (3) is -S-.

The letters A, R, and n in the formulas (5) and (6) are the same as A, R, and n in the formula (3).

The following method can also be used for production in addition to the above-described method.

EDTA dianhydride and a compound in which a group represented by the formula (3) and a hydroxyl group are bonded to each other are mixed in an organic solvent (e.g., DMF) in the presence of the basic compound at 25°C to 70°C preferably for 2 to 24 hours to produce the compound (Z). (In this case, the compound (Z) having two monovalent groups represented by the formula (3) is obtained as the main product.)

A composition of the present invention contains the compound (Z) described above.

One compound (Z) may be used or two or more compounds (Z) may be used in combination.

The composition of the present invention may be a powder, tablet, gel, sol, or solution. When the composition is a solution, it preferably contains water in addition to the compound (Z).

The composition of the present invention may further contain other components such as salts (e.g., sodium chloride) in addition to the compound (Z) and water, as long as the effects of the present invention are not inhibited.

When the composition is a solution, the concentration of the compound (Z) is preferably 1 to 500 mM, still more preferably 2 to 250 mM, based on the weight of the composition, from the viewpoint of biological tissue embrittlement.

The composition of the present invention can be used and is useful as a biological tissue embrittling agent, which is described later.

A biological tissue embrittling agent of the present invention contains the compound (Z). A preferred embodiment of the biological tissue embrittling agent of the present invention is the same as that of the composition of the present invention.

The biological tissue embrittling agent may be a powder, tablet, gel, sol, or solution. When the biological tissue embrittling agent is a solution, it preferably contains water in addition to the compound (Z).

The biological tissue embrittling agent of the present invention may further contain other components such as salts (e.g., sodium chloride) in addition to the compound (Z) and water, as long as the effects of the present invention are not inhibited.

When the biological tissue embrittling agent is a solution, the concentration of the compound (Z) is preferably 1 to 500 mM, still more preferably 2 to 250 mM, based on the weight of the biological tissue embrittling agent, from the viewpoint of biological tissue embrittlement.

The biological tissue embrittling agent may be applied to biological tissue such as gastrointestinal epithelium, mucosa, skin, or connective tissue. Biological tissue embrittlement is mainly performed as pretreatment for removing unnecessary tissue from epithelium and mucosa of a site where new cell structures are to be transplanted in regenerative medicine, and for promoting tissue regeneration by removing injured tissue and necrotic tissue. In this case, minimal invasive removal of the target tissue is required to be efficiently performed so as not to damage the underlying tissue as much as possible. From this point of view, the present inventors have successfully developed a highly functional complex that retains chelating ability while having highly safe action on biological body, based on EDTA, which is registered in the Japanese Pharmacopoeia and can be used in the medical field.

When the target is the digestive tract, the biological tissue is preferably the colonic epithelium or the small intestinal epithelium from the viewpoint of biological tissue embrittlement. It is considered that to the site where the colonic epithelium is detached and removed is transplanted various organoids (e.g., colonic organoids, small intestinal organoids).

The biological tissue embrittling agent of the present invention chelates ions such as calcium ions necessary for cell adhesion, weakens intercellular adhesion, and exerts a cell release function. Thus, the biological tissue embrittling agent may be applied to biological tissue by any process capable of bringing the agent into contact with the tissue, such as fixation, application, or retention. Also, tissue removal after tissue embrittlement may be performed by any method capable of releasing cells and connective tissue, such as detachment, abrasion, or washing. Also, cells, tissue organoids, or the like may be transplanted by any operation to the site where tissue has been embrittled and removed by the agent of the present invention. Also, the structure of cells or tissue to be transplanted may be in any form, and may be in the form of a sheet, block, or organoids.

For example, other tissues may be transplanted to the site where the colonic epithelium is detached by a method for detaching a biological tissue surface of the present invention. Thereby, properties according to the tissues transplanted can be imparted to the biological tissue. When the present technique is applied to regenerative medicine therapy for short bowel syndrome, the small intestinal organoids are preferably used (described in, for example, Non-Patent Literatures 1 and 2) .

The biological tissue embrittling agent of the present invention can also be applied to debridement that removes ulcerated, injured, or necrotic skin tissue, and can also be applied to regeneration of healthy tissue. The agent can also be applied to washing of wounds of pressure ulcers, washing of fistula tracts after surgery and anal fistulas, and detachment and washing of inflamed mucosa. In the subcutaneous tissue or body cavities, the agent can also be applied to unfavorable adhesion sites of organs and tissues as a detachment auxiliary for the purpose of promoting repair of

adhesion. Adhesion of tissues or organs during surgery may sometimes make the surgery difficult. The technique of the present invention effectively solves such a problem.

In the field of skin care, the agent of the present invention can be used as a chemical peeling auxiliary to remove old stratum corneum, effectively removing the stratum corneum while minimizing skin irritation.

The agent of the present invention can also be widely used for detachment of mucosal tissue in clinical examination. Tissue pieces have been obtained by resection, scratching, wiping, or other operation. The biological tissue embrittling agent of the present invention enables detachment of the tissue pieces from the surface of the target tissue while a certain structure in terms of cellular biochemistry is retained. Thus, the agent of the present invention can be used in tissue sampling suitable for tissue pathological diagnosis in clinical examination and three-dimensional observation in cytological examination. For example, it is useful for cervical cancer examination.

The biological tissue embrittling agent of the present invention may be in any form such as a powder, tablet, gel, sol, cream, adhesive tape, patch, or solution, as long as the agent is brought into contact with the biological tissue surface of the target site for a certain period of time. This is specifically described below in the description of the method for detaching a biological tissue surface of the present invention. A preferred exemplary technique is that gel is brought into contact with the mucosal epithelial site for a certain period of time, and thereafter, the tissue at the contact site is detached by washing or abrasion. The agent of the present invention may be brought into contact with the target site using contact equipment such as a sonde, a catheter, a syringe, a spray, an endoscope, a laparoscope, a thoracoscope, an adhesive tape, or a brush. The tissue at the contact site with which the biological tissue embrittling agent of the present invention has been in contact for a certain period of time can be removed by an additional operation such as washing, suction, or detachment.

The method for detaching a biological tissue surface of the present invention includes bringing the compound (Z) into contact with biological tissue to embrittle a surface of the biological tissue (hereinafter, sometimes abbreviated as "embrittlement step") and detaching cell tissue from the embrittled surface of the biological tissue (hereinafter sometimes abbreviated as "detachment step").

Here, in the present invention, a surface of biological tissue refers to cells bound to each other in the form of a superficial layer, and conceptually includes surfaces in organs as well as skin, gastrointestinal epithelium, and external mucosa described above.

The following describes a specific embodiment of the embrittlement step.

The compound (Z) in the biological tissue embrittling agent may be brought into contact with biological tissue, for example, in the following way: biological tissue to be embrittled (contact site) is exposed by a surgical technique (the methods disclosed in Non-Patent Literature 1 and Non-Patent Literature 2 can be used, for example); and the biological tissue embrittling agent is administered using a sonde, a catheter, a syringe, or a spray.

For example, when the biological tissue to be embrittled is the colonic epithelium, a method in which the large intestine is exposed to the outside of the abdominal cavity by a surgical technique and a biological tissue embrittling agent is administered into the intestine using a catheter may be used.

The biological tissue embrittling agent is preferably warmed to 20°C to 50°C before administration to the biological tissue to be embrittled (contact site).

The following describes a specific embodiment of the detachment step.

Cell tissue may be detached from the embrittled surface of biological tissue in the following way: the contact site is subjected to detachment by washing (detachment by the pressure of washing with physiological saline) or detachment by abrasion (detachment by abrasion with a brush such as an interdental brush). Thereby, the cell tissue is removed.

The detachment step is preferably performed promptly immediately after bringing the compound (Z) into contact with the biological tissue in the embrittlement step.

The method for detaching a biological tissue surface of the present invention may include a removal step of removing the biological tissue embrittling agent from the biological body after the detachment step.

The biological tissue embrittling agent may be removed by an operation such as washing or suction. The removal step may be performed simultaneously with detachment by washing or detachment by abrasion in the detachment step.

A biological tissue detachment kit of the present invention is a kit including the biological tissue embrittling agent of the present invention as an essential component.

The biological tissue detachment kit preferably includes in addition to the biological tissue embrittling agent of the present invention an instrument for injecting the biological tissue embrittling agent (e.g., a sonde, a catheter, a syringe, a spray) and a material used in the detachment step (e.g., physiological saline and a brush). The biological tissue detachment kit can be used for medical purposes, skin care, and the like.

EXAMPLES

The present invention will be specifically described below with reference to examples, but the present invention is not limited to these examples.

Herein, "part(s)" means "part(s) by weight".

<Production Example 1: Production of EDTA monoanhydride>

First, 148.5 parts of EDTA dianhydride (Tokyo Chemical Industry Co., Ltd.) was dissolved in 701 parts of DMF (FUJIFILM Wako Pure Chemical Corporation) at 90°C, and the solution was cooled to 75°C. Then, to the solution was added dropwise a mixture of 140.1 parts of DMF and 10.4 parts of water (1 molar equivalent relative to EDTA monoanhydride) over two hours. After the dropwise addition, the solution was mixed at 75°C for two hours to give a solid precipitate, which was suction filtered. Thus, a compound (PX-1) (EDTA monoanhydride) having a purity of 93 wt% was obtained with a yield of 62%.

<Production Example 2: Method for producing compound represented by formula (4)>

A stainless steel autoclave having stirring and temperature controlling function was charged with 275 parts of methoxypolyethylene glycol (a compound represented by the formula (4) in which the main component is that where n is 12, number average molecular weight 550, "UNIOX M-550" available from NOF Corporation) and 0.40 parts of a potassium hydroxide aqueous solution ("Super Potash" available from Toagosei Co., Ltd.). The system was purged with argon gas, depressurized, and heated to 130°C.

The contents were dehydrated at 2.7 kPa and 130°C for two hours, and then, the system was heated to 150°C. Then, 725 parts of ethylene oxide was gradually added dropwise at 145°C to 155°C over five hours while the pressure in the autoclave was controlled so as not to reach 0.4 MPa or higher. After completion of the dropwise addition, aging was performed for one hour at 145°C to 155°C until the pressure in the autoclave reached the same pressure as that at the start of the dropwise addition. Thus, 1000 parts of methoxypolyethylene glycol (a compound represented by the formula (4) in which the main component is that where n is 45) was obtained.

<Production Example 3: Method for producing compound represented by formula (4)>

A stainless steel autoclave having stirring and temperature controlling function was charged with 137 parts of methoxypolyethylene glycol (a compound represented by the formula (4) in which the main component is that where n is 12, number average molecular weight 550, "UNIOX M-550" available from NOF Corporation) and 0.4 parts of a potassium hydroxide aqueous solution ("Super Potash" available from Toagosei Co., Ltd.). The system was purged with argon gas, depressurized, and heated to 130°C.

The contents were dehydrated at 2.7 kPa and 130°C for two hours, and then, the temperature was controlled to 120°C. Then, 863 parts of ethylene oxide was gradually added dropwise at 115°C to 125°C over 10 hours while the pressure in the autoclave was controlled so as not to reach 0.4 MPa or higher. After completion of the dropwise addition, aging was performed for one hour at 115°C to 125°C until the pressure in the autoclave reached the same pressure as that at the start of the dropwise addition. Thus, 1000 parts of methoxypolyethylene glycol (a compound represented by the formula (4) in which the main component is that where n is 90) was obtained.

<Example 1: Production of compound (Z-1)>

First, 22 parts of methoxypolyethylene glycol (a compound represented by the formula (4) in which the main component is that where n is 12, number average molecular weight of 550, "UNIOX M-550" available from NOF Corporation) was dissolved in 107 parts of DMF. At 60°C, 5 parts of diisopropylethylamine (Tokyo Chemical Industry Co., Ltd.) and 11 parts of the compound (PX-1) produced in Production Example 1 were added in the stated order. The contents were mixed at 60°C for five hours, 855 parts of t-butyl methyl ether (Nacalai Tesque, Inc.) was added thereto, and the contents were suction filtered. Thus, a mixture of a compound (Z-1) (a compound (Z-1-1) (82 wt%) represented by the formula (1) containing one monovalent group represented by the formula (3) and a compound (Z-1-2) (15 wt%) represented by the formula (1) containing two monovalent groups represented by the formula (3)) and methoxypolyethylene glycol (3 wt%), which was an impurity, was obtained. The purity of the compound (Z-1) in 100 wt% of the mixture was 97 wt%. The yield of the mixture was 45%.

The main component (Z-1-1) and the minor component (Z-1-2) in the compound (Z-1) had number average molecular weights of 850 and 1400, respectively. In the compound (Z-1), the total number of moles of carboxy groups of the main component (Z-1-1) was 1.2 mmol/g based on the weight of the main component (Z-1-1) and the total number

of moles of carboxy groups of the minor component (Z-1-2) was 1.4 mmol/g based on the weight of the minor component (Z-1-2).

The number average molecular weight of the components corresponding to the compounds (Z) (the main component (Z-1-1) and the minor component (Z-1-2)) was 935. The total number of moles of carboxy groups and carboxylate groups of the components corresponding to the compounds (Z) was 1.2 mmol/g.

The compound (Z-1) had the following $^1$H-NMR spectrum chart.

$^1$H-NMR ($D_2O$, 400 MHz): 53.25 (t, 2H, $-NCH_2CH_2N-$), 3.36 (s, 3H, -OMe), 3.44 (t, 2H, $-NCH_2CH_2N-$), 3.58-3.80 (m, PEG), 3.90 (s, 4H, $-NCH_2COOH$ and $-NCH_2COO-$), 3.93 (s, 4H, $-NCH_2COOH \times 2$), 4.33 (m, 2H, $-COOCH_2CH_2O-$)

<Example 2: Production of compound (Z-2)>

First, 46 parts of methoxypolyethylene glycol (a compound represented by the formula (4) in which the main component is that where n is 12, number average molecular weight of 550, "UNIOX M-550" available from NOF Corporation) was dissolved in 104 parts of DMF. At 25°C, 10.5 parts of diisopropylethylamine and 10.5 parts of EDTA dianhydride were added in the stated order. The contents were mixed at 60°C for five hours, 829 parts of t-butyl methyl ether was added thereto, and the contents were suction filtered. Thus, a mixture of a compound (Z-2) (a compound (Z-2-2) (2 wt%) represented by the formula (1) containing one monovalent group represented by the formula (3) and a compound (Z-2-1) (95 wt%) represented by the formula (1) containing two monovalent groups represented by the formula (3)) and methoxypolyethylene glycol (3 wt%), which was an impurity, was obtained. The purity of the compound (Z-2) in 100 wt% of the mixture was 97 wt%. The yield of the mixture was 86%.

The main component (Z-2-1) and the minor component (Z-2-2) in the compound (Z-2) had number average molecular weights of 1400 and 850, respectively. In the compound (Z-2), the total number of moles of carboxy groups of the main component (Z-2-1) was 1.4 mmol/g based on the weight of the main component (Z-2-1) and the total number of moles of carboxy groups of the minor component (Z-2-2) was 1.2 mmol/g based on the weight of the minor component (Z-2-2).

The number average molecular weight of the components corresponding to the compounds (Z) (the main component (Z-2-1) and the minor component (Z-2-2)) was 1390. The total number of moles of carboxy groups and carboxylate groups of the components corresponding to the compounds (Z) was 1.4 mmol/g.

The compound (Z-2) had the following $^1$H-NMR spectrum chart.

$^1$H-NMR ($D_2O$, 400 MHz): 53.30 (s, 4H, $-NCH_2CH_2N-$), 3.31 (s, 6H, -OMe $\times$ 2), 3.50-3.86 (m, PEG), 4.02 (m, 4H, $-NCH_2COOH \times 2$), 4.35 (m, 4H, $-COOCH_2CH_2O- \times 2$)

<Example 3: Production of compound (Z-3)>

First, 23.5 parts of methoxypolyethylene glycol produced in Production Example 2 was dissolved in 110.5 parts of DMF. At 60°C, 0.5 parts of diisopropylethylamine and 3.5 parts of the compound (PX-1) produced in Production Example 1 were added in the stated order. The contents were mixed at 60°C for five hours, 862 parts of t-butyl methyl ether were added thereto, and the contents were suction filtered. Thus, a mixture of a compound (Z-3) (a compound (Z-3-1) (83 wt%) represented by the formula (1) containing one monovalent group represented by the formula (3) and a compound (Z-3-2) (11 wt%) represented by the formula (1) containing two monovalent groups represented by the formula (3)) and methoxypolyethylene glycol (6 wt%), which was an impurity, was obtained. The purity of the compound (Z-3) in 100 wt% of the mixture was 94 wt%. The yield of the mixture was 64%.

The main component (Z-3-1) and the minor component (Z-3-2) in the compound (Z-3) had number average molecular weights of 2300 and 4300, respectively. In the compound (Z-3), the total number of moles of carboxy groups of the main component (Z-3-1) was 0.4 mmol/g based on the weight of the main component (Z-3-1) and the total number of moles of carboxy groups of the minor component (Z-3-2) was 0.5 mmol/g based on the weight of the minor component (Z-3-2).

The number average molecular weight of the components corresponding to the compounds (Z) (the main component (Z-3-1) and the minor component (Z-3-2)) was 2530. The total number of moles of carboxy groups and carboxylate groups of the components corresponding to the compounds (Z) was 0.4 mmol/g.

The compound (Z-3) had the following $^1$H-NMR spectrum chart.

$^1$H-NMR ($D_2O$, 400 MHz): 53.25 (t, 2H, $-NCH_2CH_2N-$), 3.42 (s, 3H, -OMe), 3.44 (t, 2H, $-NCH_2CH_2N-$), 3.59-3.79 (m, PEG), 3.90 (s, 4H, $-NCH_2COOH$ and $-NCH_2COO-$), 3.98 (s, 4H, $-NCH_2COOH \times 2$), 4.35 (m, 2H, $-COOCH_2CH_2O-$)

<Example 4: Production of compound (Z-4)>

First, 27 parts of methoxypolyethylene glycol produced in Production Example 2 was dissolved in 126 parts of DMF. At 25°C, 2 parts of diisopropylethylamine and 2 parts of EDTA dianhydride were added in the stated order. The

contents were mixed at 60°C for five hours, 843 parts of t-butyl methyl ether were added thereto, and the contents were suction filtered. Thus, a mixture of a compound (Z-4) (a compound (Z-4-2) (1 wt%) represented by the formula (1) containing one monovalent group represented by the formula (3) and a compound (Z-4-1) (96 wt%) represented by the formula (1) containing two monovalent groups represented by the formula (3)) and methoxypolyethylene glycol (3 wt%), which was an impurity, was obtained. The purity of the compound (Z-4) in 100 wt% of the mixture was 97 wt%. The yield of the mixture was 85%.

The main component (Z-4-1) and the minor component (Z-4-2) in the compound (Z-4) had number average molecular weights of 4300 and 2300, respectively. In the compound (Z-4), the total number of moles of carboxy groups of the main component (Z-4-1) was 0.5 mmol/g based on the weight of the main component (Z-4-1) and the total number of moles of carboxy groups of the minor component (Z-4-2) was 0.4 mmol/g based on the weight of the minor component (Z-4-2).

The number average molecular weight of the components corresponding to the compounds (Z) (the main component (Z-4-1) and the minor component (Z-4-2)) was 4280. The total number of moles of carboxy groups and carboxylate groups of the components corresponding to the compounds (Z) was 0.5 mmol/g.

The compound (Z-4) had the following $^1$H-NMR spectrum chart.
$^1$H-NMR ($D_2O$, 400 MHz): 53.30 (s, 4H, $-NCH_2CH_2N-$), 3.36 (s, 6H, -OMe × 2), 3.49-3.85 (m, PEG), 4.03 (m, 4H, $-NCH_2COOH$ × 2), 4.36 (m, 4H, $-COOCH_2CH_2O-$ × 2)

<Example 5: Production of compound (Z-5)>

First, 32.3 parts of methoxypolyethylene glycol produced in Production Example 3 was dissolved in 110.9 parts of DMF. At 60°C, 1.4 parts of diisopropylethylamine and 3.4 parts of the compound (PX-1) produced in Production Example 1 were added in the stated order. The contents were mixed at 60°C for 12 hours, 852 parts of t-butyl methyl ether were added thereto, and the contents were suction filtered. Thus, a mixture of a compound (Z-5) (a compound (Z-5-1) (82 wt%) represented by the formula (1) containing one monovalent group represented by the formula (3) and a compound (Z-5-2) (15 wt%) represented by the formula (1) containing two monovalent groups represented by the formula (3)) and methoxypolyethylene glycol (3 wt%), which was an impurity, was obtained. The purity of the compound (Z-5) in 100 wt% of the mixture was 97 wt%. The yield of the mixture was 91%.

The main component (Z-5-1) and the minor component (Z-5-2) in the compound (Z-5) had number average molecular weights of 4300 and 8300, respectively. In the compound (Z-5), the total number of moles of carboxy groups of the main component (Z-5-1) was 0.23 mmol/g based on the weight of the main component (Z-5-1) and the total number of moles of carboxy groups of the minor component (Z-5-2) was 0.24 mmol/g based on the weight of the minor component (Z-5-2).

The number average molecular weight of the components corresponding to the compounds (Z) (the main component (Z-5-1) and the minor component (Z-5-2)) was 4920. The total number of moles of carboxy groups of the components corresponding to the compounds (Z) was 0.2 mmol/g.

The compound (Z-5) had the following $^1$H-NMR spectrum chart.
$^1$H-NMR ($D_2O$, 400 MHz): 53.20 (t, 2H, $-NCH_2CH_2N-$), 3.32 (s, 3H, -OMe), 3.37 (t, 2H, $-NCH_2CH_2N-$), 3.55-3.75 (m, PEG), 3.88 (s, 4H, $-NCH_2COOH$ and $-NCH_2COO-$), 3.98 (s, 4H, $-NCH_2COOH$ × 2), 4.30 (m, 2H, $-COOCH_2CH_2O-$)

The stability and chelating ability of the compounds (Z-1) to (Z-5) synthesized in Examples 1 to 5 were evaluated as follows.

<Evaluation of stability of compound (Z) (pH: 7)>

The compound (Z) was dissolved in heavy water to prepare a 1 wt% test solution of the compound (Z). (The solution had a pH of 7).

Next, the solution was allowed to stand at a temperature of 25°C, 40°C, or 60°C for three hours.

Thereafter, each solution was analyzed by $^1$H-NMR (under the conditions described below), and α was calculated as follows.

α = 100 × [(Integral of signal at around 4.3 ppm of methylene group adjacent to oxygen atom of ester bond in formula (3))/2]/[(Integral of signal at around 3.35 ppm of R (methyl group) in formula (3))/3]

Next, the percentage of retention of α before and after temperature control, which was determined by [100 ×

(α after being allowed to stand for three hours)/(α before being allowed to stand for three hours)], was calculated. Table 1 shows the results of evaluation according to the following criteria.

A percentage of retention of the ester bond derived from the formula (3) of 95% or higher ... Excellent
A percentage of retention of the ester bond derived from the formula (3) of 90% or higher to lower than 95% ... Good
A percentage of retention of the ester bond derived from the formula (3) of 80% or higher to lower than 90% ... Fair
A percentage of retention of the ester bond derived from the formula (3) of lower than 80% ... Poor

<Conditions of $^1$H-NMR evaluation>

Solvent: heavy water
Apparatus: AVANCE III HD400 (Bruker Japan Co., Ltd.)
Frequency: 400 MHz

<Evaluation of stability of compound (Z) (pH: 8)>

The compound (Z) was dissolved in heavy water, and the pH of the solution was adjusted to 8 using sodium acetate. Finally, the percentage of the weight of the compound (Z) was adjusted to 1 wt%. Thus, a test solution was obtained.
The percentage of retention of α was calculated by testing the test solution obtained above as in the <Evaluation of stability of compound (Z) (pH: 7)>, except that the test solution in <Evaluation of stability of compound (Z) (pH: 7)> was replaced by the test solution obtained above. Table 1 shows the results.

<Evaluation of stability of compound (Z) (pH: 4)>

The compound (Z) was dissolved in heavy water, and the pH of the solution was adjusted to 4 using acetic acid. Finally, the percentage of the weight of the compound (Z) was adjusted to 1 wt%. Thus, a test solution was obtained.
The percentage of retention of α was calculated by testing the test solution obtained above as in the <Evaluation of stability of compound (Z) (pH: 7)>, except that the test solution in <Evaluation of stability of compound (Z) (pH: 7)> was replaced by the test solution obtained above. Table 1 shows the results.
A higher percentage of retention of α indicates that the compound (Z) has higher stability and that production of EDTA, which causes serious side effects such as hypocalcemia, from the compound (Z) is prevented. Here, as described above, the compound (Z) of the present invention can inhibit the production of EDTA even when the pH is changed in the range of 4 and 8. This suggests that the compound (Z) of the present invention can inhibit the production of EDTA even in a pH environment in vivo.

<Evaluation of chelating ability of PEG-EDTA>

The compound (Z) and calcium chloride were mixed at a molar ratio of 1:1 and diluted with ion-exchanged water to a concentration of 10 ppm. The resulting sample was subjected to molecular weight measurement by LC/TOF-MS (under the conditions described below) to examine whether or not a signal derived from the chelate of the compound (Z) and a calcium ion was observed.
Appearance of a signal derived from the chelate of the compound (Z) and a calcium ion was evaluated as Good. Appearance of no signal derived from the chelate was evaluated as Poor. Table 1 shows the results.

<Conditions of evaluation by LC/TOF-MS>

Apparatus: available from Waters Corporation
LC: Acquity UPLC, semi-micro ultrafast LC
MS: SYNAPT HDMS, hybrid Q-TOF MS
Sample concentration: 10 ppm
Injection volume: 0.2 μL or 1.0 μL

[Table 1]

| | | | | Example | | | | |
|---|---|---|---|---|---|---|---|---|
| | | | | 1 | 2 | 3 | 4 | 5 |
| Compound (Z) | | | | (Z-1) | (Z-2) | (Z-3) | (Z-4) | (Z-5) |
| Evaluation result | Stabiity | pH 7 | 25°C | Excellent | Excellent | Excellent | Excellent | Excellent |
| | | | 40°C | Excellent | Excellent | Excellent | Excellent | Excellent |
| | | | 60°C | Excellent | Excellent | Excellent | Excellent | Excellent |
| | | pH 8 | 25°C | Excellent | Excellent | Excellent | Excellent | Excellent |
| | | | 40°C | Excellent | Excellent | Excellent | Excellent | Excellent |
| | | | 60°C | Excellent | Excellent | Excellent | Excellent | Excellent |
| | | pH 4 | 25°C | Excellent | Excellent | Excellent | Excellent | Excellent |
| | | | 40°C | Excellent | Excellent | Excellent | Excellent | Excellent |
| | | | 60°C | Excellent | Excellent | Excellent | Excellent | Excellent |
| | Chelating ability | | | Good | Good | Good | Good | Good |

<Examples 6 to 11: Production of compositions each containing compound (Z)>

Water at room temperature was added little by little to the compound (Z-1), followed by stirring and shaking with a vortex mixer (Vortex-Genie 2 available from Kennis Co., Ltd.) to prepare solutions. The water was added until the compound (Z-1) was completely dissolved. The compound (Z-1) was dissolved in about five minutes. The pH of each solution was then adjusted to 8 with 1 M NaOH. Thereafter, the solution was diluted with Hank's balanced salt solution (without Ca and Mg, with Phenol Red) (Nacalai Tesque, Cat# 17460-15) according to the molar concentrations in Table 2 to prepare compositions (C-1) to (C-6).

<Comparative Examples 1 to 7: Production of comparative compositions each containing comparative compound (Z')>

As a comparative compound (Z'-1), a composition containing disodium ethylenediaminetetraacetate ($Na_2$EDTA) was produced by the following method.

$Na_2$EDTA (0.5 mol/L EDTA solution, pH 8.0, Nacalai Tesque, Cat# 14347-21) was diluted with Hank's balanced salt solution (without Ca and Mg, with phenol red) (Nacalai Tesque, Cat# 17460-15) according to the molar concentrations in Table 2 to prepare comparative compositions (C'-1) to (C'-7).

The performance was evaluated using the compositions (C-1) to (C-6) obtained in the examples and the comparative compositions (C'-1) to (C'-7).

<Evaluation 1 of in vitro crypt isolation effect>

Eight-week-old male mice (C57B6/j, CLEA Japan, Inc.) were sacrificed by cervical dislocation and the whole large intestine was removed. The large intestine was washed with Hank's balanced salt solution (without Ca and Mg, with phenol red) (Nacalai Tesque, Cat# 17460-15). The intestinal tract was longitudinally opened and 5-mm-long tissue pieces were obtained. The tissue pieces were placed in 1.5-mL microtubes each containing 1 mL of Hank's balanced salt solution. The tissue pieces were washed by stirring for 10 minutes with a microtube mixer (MT-400 available from TOMY SEIKO CO., LTD.). After washing, the tissue pieces were transferred to other 1.5-mL microtubes each containing 1 mL of Hank's balanced salt solution, and washed in the same manner.

The tissue pieces were transferred to 1.5-mL microtubes containing the compositions (C-1) to (C-6) and the comparative compositions (C'-1) to (C'-7) each in an amount of 1 mL and were allowed to stand at 37°C for 30 minutes.

After 30 minutes, the tissue pieces were transferred to other 1.5-mL microtubes each containing 1 mL of Hank's balanced salt solution and stirred for five minutes with a microtube mixer. Thereby, the crypts were isolated. After five minutes, to the microtubes containing the tissue was added 0.5 mL of a 4% paraformaldehyde/phosphate buffer (Nacalai Tesque, Cat# 09154-85). Thereby, the tissue was fixed. A 50-uL portion was sampled therefrom and dropped onto

dishes, and the number of crypts contained was counted using an optical microscope. The number of crypts was counted three times for each concentration, and the average was calculated therefrom. Table 2 shows the results.

The presence of one or more crypts was considered that a sufficient embrittlement effect was obtained.

[Table 2]

| | Example | | | | | | Comparative Example | | | | | | |
|---|---|---|---|---|---|---|---|---|---|---|---|---|---|
| | 6 | 7 | 8 | 9 | 10 | 11 | 1 | 2 | 3 | 4 | 5 | 6 | 7 |
| Composition (C) | (C-1) | (C-2) | (C-3) | (C-4) | (C-5) | (C-6) | (C'-1) | (C-2) | (C-3) | (C-4) | (C-5) | (C-6) | (C-7) |
| Type of compound (Z) | (Z-1) | | | | | | (Z'-1) | | | | | | |
| Concentration (mM) of compound (Z) or compound (Z') | 2 | 8 | 16 | 32 | 64 | 128 | 0 | 2 | 8 | 16 | 32 | 64 | 128 |
| Number of crypts | 6 | 38.33 | 172.67 | 221 | 166 | 107 | 0 | 48.67 | 156.33 | 140 | 129.33 | 115.33 | 95.67 |

<Examples 12 to 19: Production of compositions each containing compound (Z)>

Water at room temperature was added little by little to each of the compounds (Z) shown in Table 3, followed by stirring and shaking with a vortex mixer (Vortex-Genie 2 available from KENIS LIMITED) to prepare solutions. The water was added until each compound (Z) was completely dissolved. The compound (Z) was dissolved in about five minutes. The pH of each solution was then adjusted to 8 with 1 M NaOH. Thereafter, the solution was diluted with Hank's balanced salt solution (without Ca and Mg, with Phenol Red) (Nacalai Tesque, Cat# 17460-15) according to the molar concentrations in Table 3 to prepare compositions (C-7) to (C-14).

<Comparative Examples 8 and 9: Production of comparative compositions each containing comparative compound (Z')>

As a comparative compound (Z'-1), a composition containing disodium ethylenediaminetetraacetate ($Na_2EDTA$) was produced by the following method.

$Na_2EDTA$ (0.5 EDTA solution, pH 8.0, Nacalai Tesque, Cat# 14347-21) was diluted with Hank's balanced salt solution (without Ca and Mg, with phenol red) (Nacalai Tesque, Cat# 17460-15) according to the molar concentrations in Table 3 to prepare comparative compositions (C'-8) and (C'-9).

The performance was evaluated using the compositions (C-7) to (C-14) obtained in the examples and the comparative compositions (C'-8) and (C'-9).

<Evaluation 2 of in vitro crypt isolation effect>

Eight-week-old male mice (C57B6/j, CLEA Japan, Inc.) were sacrificed by cervical dislocation and a 4-cm long piece of the distal large intestine was removed. The large intestine was washed with Hank's balanced salt solution (without Ca and Mg, with phenol red) (Nacalai Tesque, Cat# 17460-15). The intestinal tract was longitudinally opened and minced using a razor blade (Feather Cat# FAS-10). The minced tissue was suspended in 40 mL of Hank's balanced salt solution, and the solution was divided into equal 4-mL portions in 15-mL tubes. The minced tissue was precipitated by centrifugation at 1500 rpm for one minute and the supernatant was discharged. The minced tissue was suspended to 1 mL of Hank's balanced salt solution, and the minced tissue together with the solution was transferred to other 1.5 mL microtubes. The minced tissue was washed by stirring for 10 minutes with a microtube mixer (MT-400 available from TOMY SEIKO CO., LTD.). The minced tissue was precipitated by centrifugation of the tubes at 15000 rpm for one minute, and the supernatant was discharged. To the minced tissue was added 1 mL of Hank's balanced salt solution, and the minced tissue was washed in the same manner. The minced tissue was precipitated by centrifugation of the tubes at 15000 rpm for one minute, and the supernatant was discharged. To the tubes were added the compositions (C-7) to (C-14) and the comparative compositions (C'-8) and (C'-9) each in an amount of 1 ml, and the tubes were allowed to stand at 37°C for 30 minutes.

After 30 minutes, the minced tissue was precipitated by centrifugation of the tubes at 15000 rpm for one minute, and the supernatant was discharged. To the tubes was added 1 mL of Hank's balanced salt solution, and the solution was stirred for five minutes with a microtube mixer. Thereby, the crypts were isolated. After five minutes, to the microtubes containing the tissue was added 0.5 mL of a 4% paraformaldehyde/phosphate buffer (Nacalai Tesque, Cat# 09154-85). Thereby, the tissue was fixed. A 50-uL portion was sampled therefrom and dropped onto dishes, and the number of crypts contained was counted using an optical microscope. The number of crypts was counted three times for each concentration, and the average was calculated therefrom. This experiment was performed six times, and the average was calculated therefrom. Table 3 shows the results.

The presence of one or more crypts indicated that a sufficient embrittlement effect was obtained.

[Table 3]

| | Example | | | | | | | | Comparative Example | |
|---|---|---|---|---|---|---|---|---|---|---|
| | 12 | 13 | 14 | 15 | 16 | 17 | 18 | 19 | 8 | 9 |
| Composition (C) | (C-7) | (C-8) | (C-9) | (C-10) | (C-11) | (C-12) | (C-13) | (C-14) | (C'-8) | (C'-9) |
| Type of compound (Z) | (Z-1) | (Z-3) | (Z-5) | (Z-4) | (Z-1) | (Z-3) | (Z-5) | (Z-4) | (Z'-1) | (Z'-1) |

(continued)

| | Example | | | | | | | | Comparative Example | |
|---|---|---|---|---|---|---|---|---|---|---|
| | 12 | 13 | 14 | 15 | 16 | 17 | 18 | 19 | 8 | 9 |
| Concentration (mM) of compound (Z) or compound (Z') | 3 | 3 | 3 | 3 | 10 | 10 | 10 | 10 | 3 | 10 |
| Number of crypts | 55.67 | 71.61 | 47.61 | 19.39 | 103.56 | 88.78 | 72.39 | 71.28 | 108.89 | 169.56 |

<Examples 30 to 24: Production of biological tissue embrittling agents>

Water at room temperature was added little by little to each of the compounds (Z) shown in Table 4, followed by stirring and shaking with a vortex mixer (Vortex-Genie 2 available from Kennis Co., Ltd.) to prepare solutions. The water was added until each compound (Z) was completely dissolved. The compound was dissolved in about five minutes. The pH of each solution was then adjusted to 8 with 1 M NaOH. Thereby, biological tissue embrittling agents (M-1) to (M-5) were obtained. The concentrations of the compounds (Z) in the biological tissue embrittling agents (M) were as shown in Table 4.

<Comparative Examples 10 to 12: Production of comparative biological tissue embrittling agents>

As a comparative compound (Z'-1), a biological tissue embrittling agent containing disodium ethylenediamine-tetraacetate ($Na_2EDTA$) was produced.

$Na_2EDTA$ (0.5 mol/L EDTA solution, pH 8.0, Nacalai Tesque, Cat# 14347-21) was diluted with water according to the molar concentrations shown in Table 4 to prepare comparative biological tissue embrittling agents (M'-1) to (M'-3) .

The biological tissue embrittling agents (M-1) to (M-5) obtained in the examples and the comparative biological tissue embrittling agents (M'-1) to (M'-3) were used, and the performance was evaluated by the following method.

<Evaluation 1 of in vivo epithelial detachment effect>

Eight-week-old male mice (C57B6/j, CLEA Japan, Inc.) were anesthetized with isoflurane (FUJIFILM Cat# 099-06571) and fixed in the supine position. A longitudinal 1-cm abdominal midline incision was made, and the proximal large intestine was exposed to the outside of the abdominal cavity. A small incision was made in the proximal large intestine 1 cm anal side from the cecum, and a catheter was inserted toward the anal. The lumen was washed with Dulbecco's phosphate buffered saline (without Ca and Mg) (Nacalai Tesque, Cat# 14249-24). A small incision was made 1 cm anal side from the catheter insertion site, and another catheter was inserted toward the oral side. These two catheters allowed perfusion of the 1-cm-long intestinal tract. The catheters were each fixed with a thread from the outside of the intestinal tract, together with the vasculature, to cut off the blood flow in the 1-cm-long intestinal tract sandwiched between the two catheters. The biological tissue embrittling agent (M-1) and the comparative biological tissue embrittling agents (M'-1) to (M'-3) each in an amount of 8 mL warmed to 50°C were injected from the catheter over two minutes.

Immediately after the injection of the biological tissue embrittling agents, 25 mL of Dulbecco's phosphate-buffered saline (without Ca and Mg) was continuously injected three times each over 45 seconds, whereby the crypts were detached. Then, 20 mL of Dulbecco's Modified Eagle's Medium, high glucose, (Sigma-Aldrich Cat# D5796) was injected to antagonize the biological tissue embrittling agents. The treated proximal large intestine was excised, and the tissue was fixed with a 4% paraformaldehyde/phosphate buffer. The large intestine was longitudinally cut and flat opened, and was imaged with a fluorescent stereo microscope (Leica M165 FC) and an all-in-one fluorescence microscope (KEYENCE BZ-X800). The images were analyzed with ImageJ and the detached area was measured. Table 4 shows the results.

<Evaluation 2 of in vivo epithelial detachment effect>

Whether or not the detachment effect can be expanded by extending the administration time of PEG-EDTA was evaluated as follows: the detached area was measured as in <Evaluation 1 of in vivo epithelial detachment effect>, except that the operation in which the biological tissue embrittlement agents (M-1) to (M-5) each in an amount of 8 mL

warmed to 50°C were injected from the catheter over two minutes was repeated four times instead of once. Table 4 shows the results.

Confirmation of epithelial detachment is regarded as achievement of a sufficient embrittlement effect. (In this evaluation, 1 $mm^2$ or more of epithelial detachment is preferably confirmed.)

[Table 4]

| | Example | | | | | Comparative | | |
|---|---|---|---|---|---|---|---|---|
| | 20 | 21 | 22 | 23 | 24 | 10 | 11 | 12 |
| Biological tissue embrittling agent (M) | (M-1) | (M-2) | (M-3) | (M-4) | (M-5) | (M'-1) | (M'-2) | (M'-3) |
| Type of compound (Z) | (Z-1) | (Z-3) | (Z-4) | (Z-4) | (Z-4) | (Z'-1) | (Z'-1) | (Z'-1) |
| Concentration (mM) of compound (Z) or compound (Z') | 230 | 138 | 125 | 62.5 | 2 | 0 | 230 | 500 |
| Evaluation 1 of epithelial detachment effect Detached area ($mm^2$) | 8.4 | - | - | - | - | 0 | 30.2 | 66.0 |
| Evaluation 2 of epithelial detachment effect Detached area ($mm^2$) | 71.2 | 13.7 | 1.7 | 12.3 | 10.6 | - | - | - |

<Evaluation examples and comparative evaluation examples: Toxicity evaluation by oral administration>

Eight-week-old male mice (C57B6/j, CLEA Japan, Inc.) were fasted from 20:00 on the day before administration (drinking water allowed.) After 12 hours. administration was started at 8:00 on the examination day. For preparation of solutions for administration, each mouse was corresponding to LD50 of $N_{a2}$EDTA (see Initial risk assesment method for chemical substances (provisional version), No. 14., Act on Confirmation, etc. of Release Amounts of Specific Chemical Substances in the Environment and Promotion of Improvements to the Management Thereof, Cabinet Order: 1-47, CAS No.: 60-00-4), half the above amount (0.5 times LD50), and 1.5 times the above amount (1.5 times LD50) were calculated.

An aqueous solution (1 ml) containing $Na_2$EDTA (0.5 mol/L EDTA solution, pH 8.0, Nacalai Tesque, Cat# 14347-21) in an amount corresponding to its LD50 and an aqueous solution (1 ml) containing the $Na_2$EDTA in an amount half the above amount (0.5 times LD50) were prepared and used as toxicity evaluation reagents (P'-1) and (P'-2), respectively.

Separately, an aqueous solution (1 mL) containing the same mole number of the compound (Z-1) as that of $Na_2$EDTA in the toxicity evaluation reagent (P'-1) was prepared and used as a toxicity evaluation reagent (P-1). In addition, toxicity evaluation reagents (P-3), (P-4), and (P-5) were prepared using reagents using (Z-3), (Z-5), and (Z-4), respectively, instead of (Z-1).

An aqueous solution (1 mL) containing the same mole number of the compound (Z-1) as that of $Na_2$EDTA in the toxicity evaluation reagent (P'-2) was prepared and used as a toxicity evaluation reagent (P-2).

An aqueous solution (1 mL) containing the compound (Z-3) in an amount 1.5 times the mole number of $Na_2$EDTA (1.5 times LD50) in the toxicity evaluation reagent (P'-1) was prepared and used as a toxicity evaluation reagent (P-6) .

Next, the toxicity evaluation reagents (P-1) to (P-6) and the toxicity evaluation reagents (P'-1) and (P'-2) were separately administered to the mice.

At the administration, the mice were anesthetized with isoflurane and the toxicity evaluation reagents were forcibly orally administered using a disposable oral sonde (FUCHIGAMI Cat# 4200). For confirmation, three mice were used for each solution containing an amount corresponding to LD50, five mice were used for each solution containing 0.5 times LD50, and five mice were used for each solution containing 1.5 times LD50. Evaluation was performed according to the following criteria. Tables 5 to 7 show the results.

Excellent: Mice were alive at least a week after full-dose administration.

Good: Mice were alive at least 15 minutes after full-dose administration.

Poor: Mice died in less than 15 minutes after full-dose administration.

[Table 5]

| | Evaluation Example 1 | Evaluation Example 3 | Evaluation Example 4 | Evaluation Example 5 | Comparative Evaluation Example 1 |
|---|---|---|---|---|---|
| Toxicity evaluation reagent (P) | (P-1) | (P-3) | (P-4) | (P-5) | (P'-1) |
| Type of compound | (Z-1) | (Z-3) | (Z-5) | (Z-4) | (Z'-1) |
| Amount of compound (Mole number) | Same mole number as LD50 of $Na_2EDTA$ | | | | Same mole number as LD50 of $Na_2EDTA$ |
| Toxicity evaluation (Status of three animals) | Good | Excellent | Excellent | Excellent | Poor |
| | Good | Excellent | Excellent | Excellent | Poor |
| | Good | Excellent | Good | Excellent | Poor |

[Table 6]

| | Evaluation Example 2 | Comparative Evaluation Example 2 |
|---|---|---|
| Toxicity evaluation reagent (P) | (P-2) | (P'-2) |
| Type of compound | (Z-1) | (Z'-1) |
| Amount of compound (Mole number) | Mole number 0.5 times LD50 of $Na_2EDTA$ | Mole number 0.5 times LD50 of $Na_2EDTA$ |
| Toxicity evaluation (Status of five animals) | Excellent | Excellent |
| | Excellent | Excellent |
| | Excellent | Excellent |
| | Excellent | Poor |
| | Excellent | Poor |

[Table 7]

| | Evaluation Example 6 |
|---|---|
| Toxicity evaluation reagent (P) | (P-6) |
| Type of compound | (Z-3) |
| Amount of compound (Mole number) | Mole number 1.5 times LD50 of $Na_2EDTA$ |
| Toxicity evaluation (Status of five animals) | Excellent |
| | Excellent |
| | Excellent |
| | Good |
| | Good |

<Evaluation Examples 7 to 25 and Comparative Evaluation Examples 3 to 5: Toxicity evaluation by intraperitoneal administration>

Eight-week-old male mice (C57B6/j, CLEA Japan, Inc.) were administered as follows. For preparation of solutions for administration, each mouse was weighed immediately before administration, and an amount corresponding to LD50 of $Na_2EDTA$ (see Initial risk assessment method for chemical substances (provisional version), No. 14., Act on

Confirmation, etc. of Release Amounts of Specific Chemical Substances in the Environment and Promotion of Improvements to the Management Thereof, Cabinet Order: 1-47, CAS No.: 60-00-4), half the above amount (0.5 times LD50), 1.5 times the above amount (1.5 times LD50), 2.0 times the amount above (2.0 times LD50), 3.0 times the above amount (3.0 times LD50), 4.0 times the above amount (4.0 times LD50), and 8.0 times the above amount (8.0 times LD50) were calculated.

An aqueous solution (0.5 ml) containing $Na_2EDTA$ (0.5 mol/L EDTA solution, pH 8.0, Nacalai Tesque, Cat# 14347-21) in an amount corresponding to its LD50, an aqueous solution (0.5 mL) containing the $Na_2EDTA$ in an amount half the above amount (0.5 times LD50), and an aqueous solution (0.5 mL) containing the $Na_2EDTA$ in an amount 1.5 times the above amount (1.5 times LD50) were prepared and used as toxicity evaluation reagents (P'-3) and (P'-5), respectively.

Separately, an aqueous solution (0.5 mL) containing the same mole number of the compound (Z-1) as that of $Na_2EDTA$ in the toxicity evaluation reagent (P'-3), an aqueous solution (0.5 mL) containing the same mole number of the compound (Z-1) as that of $Na_2EDTA$ in the toxicity evaluation reagent (P'-4), and an aqueous solution (0.5 mL) containing the same mole number of the compound (Z-1) as that of $Na_2EDTA$ in the toxicity evaluation reagent (P'-5) were prepared and used as toxicity evaluation reagents (P-7) to (P-9), respectively.

An aqueous solution (0.5 mL) containing the compound (Z-3) in an amount 1.5 times the mole number of $Na_2EDTA$ (1.5 times LD50) in the toxicity evaluation reagent (P'-3) was prepared and used as a toxicity evaluation reagent (P-10). In addition, toxicity evaluation reagents (P-11) and (P-12) were prepared using (Z-5) and (Z-4), respectively, instead of (Z-3) .

An aqueous solution (0.5 mL) containing the compound (Z-1) in an amount 2.0 times the mole number of $Na_2EDTA$ (2.0 times LD50) in the toxicity evaluation reagent (P'-3) was prepared and used as a toxicity evaluation reagent (P-13). In addition, toxicity evaluation reagents (P-14), (P-15), and (P-16) were prepared using (Z-3), (Z-5), and (Z-4), respectively, instead of (Z-1).

An aqueous solution (0.5 mL) containing the compound (Z-3) in an amount 3.0 times the mole number of $Na_2EDTA$ (3.0 times LD50) in the toxicity evaluation reagent (P'-3) was prepared and used as a toxicity evaluation reagent (P-17). In addition, toxicity evaluation reagents (P-18) and (P-19) were prepared using (Z-5) and (Z-4), respectively, instead of (Z-3) .

An aqueous solution (0.5 mL) containing the compound (Z-3) in an amount 4.0 times the mole number of $Na_2EDTA$ (4.0 times LD50) in the toxicity evaluation reagent (P'-3) was prepared and used as a toxicity evaluation reagent (P-20). In addition, toxicity evaluation reagents (P-21) and (P-22) were prepared using (Z-5) and (Z-4), respectively, instead of (Z-3) .

An aqueous solution (1.5 mL) containing the compound (Z-3) in an amount 8.0 times the mole number of $Na_2EDTA$ (8.0 times LD50) in the toxicity evaluation reagent (P'-3) was prepared and used as a toxicity evaluation reagent (P-23). In addition, toxicity evaluation reagents (P-24) and (P-25) were prepared using (Z-5) and (Z-4), respectively, instead of (Z-3) .

Next, the toxicity evaluation reagents (P-7) to (P-25) and the toxicity evaluation reagents (P'-3) and (P'-5) were separately administered to the mice.

At the administration, the mice were anesthetized with isoflurane and the toxicity evaluation regents were separately administered intraperitoneally using a syringe equipped with a needle. A 1-ml syringe (Terumo Cat# SS-10M2913A) with a 29G needle was used for each aqueous solution (0.5 ml) containing a compound in an amount 0.5 to 4.0 times the mole number of $Na_2EDTA$, and a 2.5-ml syringe (Terumo Cat #SS-02SZ) with a 27G needle (Terumo Cat #NN-2719S) was used for each aqueous solution (1.5 ml) containing a compound in an amount 0.5 to 4.0 times the mole number of $Na_2EDTA$. For confirmation, three mice were used for each solution containing an amount corresponding to LD50, for each solution containing 0.5 times LD50, for each solution containing 1.5 times LD50, for each solution containing 2.0 times LD50, and for each solution containing 3.0 times LD50, and two mice were used for each solution containing 4.0 times LD50 and for each solution containing 8.0 times LD50. Evaluation was performed according to the following criteria. Tables 8 to 14 show the results.

Excellent: Mice were alive at least a week after full-dose administration.

Good: Mice were alive at least 60 minutes after full-dose administration.

Fair: Mice were alive at least 15 minutes after full-dose administration.

Poor: Mice died in less than 15 minutes after full-dose administration.

[Table 8]

| | Evaluation Example 7 | Com parative Evaluation Example 3 |
|---|---|---|
| Toxicity evaluation reagent (P) | (P-7) | (P'-3) |
| Type of compound | (Z-1) | (Z-1) |

(continued)

| | Evaluation Example 7 | Com parative Evaluation Example 3 |
|---|---|---|
| Amount of compound (Mole number) | Same mole number as LD50 of $Na_2EDTA$ | Same mole number as LD50 of $Na_2EDTA$ |
| Toxicity evaluation (Status of three animals) | Excellent | Excellent |
| | Excellent | Fair |
| | Excellent | Poor |

[Table 9]

| | Evaluation Example 8 | Comparative Evaluation Example 4 |
|---|---|---|
| Toxicity evaluation reagent (P) | (P-8) | (P'-4) |
| Type of compound | (Z-1) | (Z'-1) |
| Amount of compound (Mole number) | Mole number 0.5 times LD50 of $Na_2EDTA$ | Mole number 0.5 times LD50 of $Na_2EDTA$ |
| Toxicity evaluation (Status of three animals) | Excellent | Excellent |
| | Excellent | Excellent |
| | Excellent | Excellent |

[Table 10]

| | Evaluation Example 9 | Evaluation Example 10 | Evaluation Example 11 | Evaluation Example 12 | Comparative Evaluation Example 5 |
|---|---|---|---|---|---|
| Toxicity evaluation reagent (P) | (P-9) | (P-10) | (P-11) | (P-12) | (P'-5) |
| Type of compound | (Z-1) | (Z-3) | (Z-5) | (Z-4) | (Z'-1) |
| Amount of compound (Mole number) | Mole number 1.5 times LD50 of Na2EDTA | | | | Mole number 1.5 times LD50 of Na2EDTA |
| Toxicity evaluation (Status of three animals) | Excellent | Excellent | Excellent | Excellent | Poor |
| | Excellent | Excellent | Excellent | Excellent | Poor |
| | Excellent | Excellent | Excellent | Excellent | Poor |

[Table 11]

| | Evaluation Example 13 | Evaluation Example 14 | Evaluation Example 15 | Evaluation Example 16 |
|---|---|---|---|---|
| Toxicity evaluation reagent (P) | (P-13) | (P-14) | (P-15) | (P-16) |
| Type of compound | (Z-1) | (Z-3) | (Z-5) | (Z-4) |
| Amount of compound (Mole number) | Mole number 2.0 times LD50 of $Na_2EDTA$ | | | |

(continued)

| | Evaluation Example 13 | Evaluation Example 14 | Evaluation Example 15 | Evaluation Example 16 |
|---|---|---|---|---|
| Toxicity evaluation (Status of three animals) | Excellent | Excellent | Excellent | Excellent |
| | Poor | Excellent | Excellent | Excellent |
| | Poor | Excellent | Good | Excellent |

[Table 12]

| | Evaluation Example 17 | Evaluation Example 18 | Evaluation Example 19 |
|---|---|---|---|
| Toxicity evaluation reagent (P) | (P-17) | (P-18) | (P-19) |
| Type of compound | (Z-3) | (Z-5) | (Z-4) |
| Amount of compound (Mole number) | Mole number 3.0 times LD50 of Na2EDTA | | |
| Toxicity evaluation (Status of three animals) | Excellent | Excellent | Excellent |
| | Excellent | Good | Excellent |
| | Excellent | Good | Excellent |

[Table 13]

| | Evaluation Example 20 | Evaluation Example 21 | Evaluation Example 22 |
|---|---|---|---|
| Toxicity evaluation reagent (P) | (P-20) | (P-21) | (P-22) |
| Type of compound | (Z-3) | (Z-5) | (Z-4) |
| Amount of compound (Mole number) | Mole number 4.0 times LD50 of Na2EDTA | | |
| Toxicity evaluation (Status of two animals) | Fair | Good | Good |
| | Fair | Good | Good |

[Table 14]

| | Evaluation Example 23 | Evaluation Example 24 | Evaluation Example 25 |
|---|---|---|---|
| Toxicity evaluation reagent (P) | (P-23) | (P-24) | (P-25) |
| Type of compound | (Z-3) | (Z-5) | (Z-4) |
| Amount of compound (Mole number) | Mole number 8.0 times LD50 of Na2EDTA | | |
| Toxicity evaluation (Status of two animals) | Fair | Fair | Good |
| | Fair | Fair | Good |

<Evaluation Examples 26 to 29 and Comparative Evaluation Examples 6 and 7: Evaluation of Ca concentration after intraperitoneal administration>

An aqueous solution (0.5 ml) containing $Na_2EDTA$ (0.5 mol/L EDTA solution, pH 8.0, Nacalai Tesque, Cat# 14347-21) in an amount 2.0 times the amount corresponding to its LD50 (2.0 times LD50) was prepared as in "Toxicity evaluation by intraperitoneal administration" and used as a toxicity evaluation reagent (P'-6).

An aqueous solution (0.5 mL) containing the same mole number of the compound (Z-1) as that of $Na_2EDTA$ in the toxicity evaluation reagent (P'-6) was prepared and used as a toxicity evaluation reagent (P-26).

In addition, toxicity evaluation reagents (P-27), (P-28), and (P-29) were prepared using (Z-3), (Z-5), and (Z-4), respectively, instead of (Z-1).

The aqueous solutions described above were intraperitoneally administered to the mice as in "Toxicity evaluation by intraperitoneal administration", and blood was collected two minutes later. Serum was obtained from the collected blood, and the corrected Ca concentration (mg/dL) (the concentration of Ca, in the blood, excluding Ca bound to blood albumin) was determined based on the following equation:

$$\text{Corrected Ca concentration (mg/dL)} = \text{Measured Ca concentration (mg/dL)} + 4 - \text{Alb (g/dL)}$$

In the equation, "Measured Ca concentration (mg/dL)" and "Alb (g/dL)" are values obtained by the following measurements.

Measured Ca concentration (mg/dL): The Ca concentration (mg/dL) (Measured Ca concentration) of the serum obtained from the collected blood was measured using the Arsenazo III method (outsourced to BML Inc.).

Alb (g/dL): The albumin concentration (g/dL) of the serum obtained from the collected blood was measured using Albumin IIHA-Test Wako (FUJIFILM Wako Pure Chemical Corporation). (The measurement was outsourced to FUJIFILM Wako Pure Chemical Corporation).

Separately, water was administered instead of the toxicity evaluation reagent, and a corrected Ca concentration (mg/dL) was measured in the same manner as above. The water used as a toxicity evaluation reagent was represented by (P'-7).

Three mice were used for each toxicity evaluation reagent, and the mean value of the corrected Ca concentrations (mg/dL) was obtained. Table 15 shows the results.

From the viewpoint of safety in biological body, the corrected Ca concentration is preferably 7.0 mg/dL or more. When the corrected Ca concentration is less than 7.0 mg/dL, symptoms such as neurological symptoms (e.g., tetany and generalized convulsions) and severe arrhythmias (e.g., QT prolongation and bradycardia) may occur.

[Table 15]

| | Evaluation Example 26 | Evaluation Example 27 | Evaluation Example 28 | Evaluation Example 29 | Comparative Evaluation Example 6 | Comparative Evaluation Example 7 |
|---|---|---|---|---|---|---|
| Toxicity evaluation reagent (P) | (P-26) | (P-27) | (P-28) | (P-29) | (P'-6) | (P'-7) |
| Type of compound (Z) | (Z-1) | (Z-3) | (Z-5) | (Z-4) | (Z'-1) | Water |
| Evaluation result (mg/dL) | 8.43 | 9.33 | 10.07 | 9.6 | 3.7 | 10.03 |

INDUSTRIAL APPLICABILITY

Use of compositions containing the compounds of the present invention as biological tissue embrittling agents exhibits the same tissue embrittlement effect as conventional EDTA and exhibits less toxicity to biological body. Thus, the compositions described above are useful as biological tissue embrittling agents.

**Claims**

**1.** A compound (Z) represented by the formula (1) or (2):

[Chem. 1]

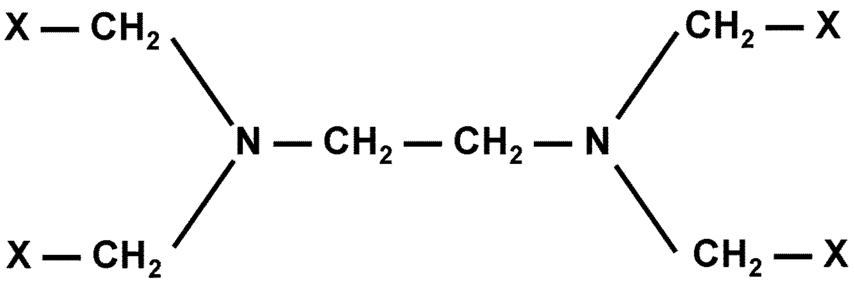

(1)

[Chem. 2]

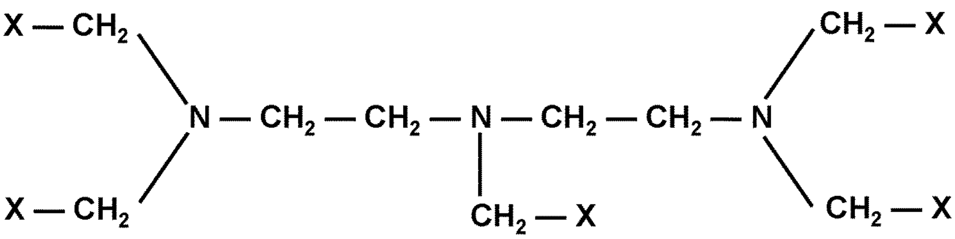

(2)

wherein Xs in each of the formulas (1) and (2) are each independently a carboxy group, a carboxylate group, or a monovalent group represented by the formula (3); at least one of Xs in each of the formulas (1) and (2) is a carboxy group or a carboxylate group; and at least one of Xs in each of the formulas (1) and (2) is a monovalent group represented by the formula (3):

$$-C(O)-Y-(AO)_n-R \qquad (3)$$

wherein -Y- is -O-, -NH-, or -S-; A is a C2-C4 alkylene group; R is a hydrogen atom or a C1-C15 monovalent hydrocarbon group in which a hydrogen atom is optionally substituted with a C1-C10 alkoxy group; n is an integer of 4 to 1000; at least one of n As is an ethylene group; and when the formulas (1) and (2) each have multiple monovalent groups represented by the formula (3), each of -Y-, A, R, and n is the same or different among the monovalent groups.

**2.** A composition comprising the compound (Z) according to claim 1.

**3.** A biological tissue embrittling agent comprising the compound (Z) according to claim 1.

**4.** The biological tissue embrittling agent according to claim 3, wherein the biological tissue is gastrointestinal epithelium, mucosa, skin, or connective tissue.

**5.** A method for detaching a biological tissue surface, comprising:

bringing the compound (Z) according to claim 1 into contact with biological tissue to embrittle a surface of the biological tissue; and
detaching cell tissue from the embrittled surface of the biological tissue.

**6.** The method for detaching a biological tissue surface according to claim 5, wherein the biological tissue is gastrointestinal epithelium, mucosa, skin, or connective tissue.

**7.** A biological tissue detachment kit comprising the biological tissue embrittling agent according to claim 3.

## INTERNATIONAL SEARCH REPORT

International application No.
**PCT/JP2021/047282**

**A. CLASSIFICATION OF SUBJECT MATTER**

***C08G 65/333***(2006.01)i; ***A61K 31/197***(2006.01)i; ***A61K 31/77***(2006.01)i; ***A61P 1/04***(2006.01)i; ***A61P 41/00***(2006.01)i; ***C08L 71/02***(2006.01)i
FI: C08G65/333; A61K31/197; A61K31/77; A61P1/04; A61P41/00; C08L71/02

According to International Patent Classification (IPC) or to both national classification and IPC

**B. FIELDS SEARCHED**

Minimum documentation searched (classification system followed by classification symbols)

C08G2/00-85/00; C08L1/00-101/16; A61K31/197; A61K31/77; A61P1/04; A61P41/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 6-511033 A (UNGER, Evan C.) 08 December 1994 (1994-12-08)<br>claims, column "examples" (for example, example 1), etc. | 1-2<br>3-7 |
| X<br>A | JP 9-511013 A (NYCOMED IMAGING A.S) 04 November 1997 (1997-11-04)<br>claims, column "examples", etc. | 1-2<br>3-7 |
| X<br>A | JP 2007-533624 A (BASF AKTIENGESELLSCHAFT) 22 November 2007 (2007-11-22)<br>claims (in particular, claim 12), column "examples", etc. | 1-2<br>3-7 |
| X<br>A | JP 2008-508346 A (BASF SE) 21 March 2008 (2008-03-21)<br>claims, paragraph [0056], formula 7 (compound VI), etc. | 1-2<br>3-7 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 March 2022** | **22 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
**PCT/JP2021/047282**

C. DOCUMENTS CONSIDERED TO BE RELEVANT

| Category* | Citation of document, with indication, where appropriate, of the relevant passages | Relevant to claim No. |
|---|---|---|
| X | US 6020373 A (EASTERN VIRGINIA MEDICAL SCHOOL) 01 February 2000 (2000-02-01) claims, column 5, structural formula, etc. | 1-2 |
| A | | 3-7 |
| A | US 2008/0197082 A1 (BASF AKTIENGESELLSCHAFT) 21 August 2008 (2008-08-21) | 1-7 |

Form PCT/ISA/210 (second sheet) (January 2015)

**INTERNATIONAL SEARCH REPORT**
**Information on patent family members**

International application No.
**PCT/JP2021/047282**

| Patent document cited in search report | Publication date (day/month/year) | Patent family member(s) | Publication date (day/month/year) |
|---|---|---|---|
| JP 6-511033 A | 08 December 1994 | US 5385719 A<br>claims, column "examples" (for example, example 1), etc.<br>WO 1993/006148 A1 | |
| JP 9-511013 A | 04 November 1997 | US 5730968 A<br>claims, column "examples", etc.<br>WO 1995/026754 A1 | |
| JP 2007-533624 A | 22 November 2007 | US 2006/0287548 A1<br>claims (in particular, claim 10), column "examples", etc.<br>WO 2005/030907 A1<br>EP 1518916 A1 | |
| JP 2008-508346 A | 21 March 2008 | US 2008/0312432 A1<br>claims, paragraph [0081], compound VI, etc.<br>WO 2006/015799 A1 | |
| US 6020373 A | 01 February 2000 | WO 1997/014413 A1 | |
| US 2008/0197082 A1 | 21 August 2008 | WO 2006/134145 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Genes and Development,* 2014, vol. 28, 1752-1757
- *Cell Stem Cell,* 2018, vol. 22, 1-6
- *Nature,* 01 April 2021, vol. 592, 99-104
- *CHEMICAL ABSTRACTS,* 60-00-4